# EUROPEAN PATENT SPECIFICATION

(11) **EP 2 329 267 B1**
(45) Date of publication and mention of the grant of the patent: **17.07.2013**
(21) Application number: 09781570.8
(22) Date of filing: 06.08.2009
(51) Int. Cl.: G01N 33/53, C07K 16/44, C07K 16/40

(54) **MANNOSE-6-PHOSPHATE-BINDING ANTIBODIES AND THEIR USES**
MANNOSE-6-PHOSPHAT BINDENDE ANTIKÖRPER UND DEREN VERWENDUNGEN
ANTICORPS DE LIAISON AU MANNOSE-6-PHOSPHATE ET LEURS UTILISATIONS

(30) Priority: 08.08.2008 EP 08162125
(43) Date of publication of application: 08.06.2011
(73) Proprietor: Universitätsklinikum Hamburg-Eppendorf, 20246 Hamburg (DE); Forschungszentrum Borstel, 23845 Borstel (DE)
(72) Inventor: BRAULKE, Thomas, 22529 Hamburg (DE); MÜLLER-LOENNIES, Sven, 23564 Lübeck (DE)
(74) Representative: UEXKÜLL & STOLBERG
(86) International application number: PCT/EP2009/060224
(87) International publication number: WO 2010/015683

(56) References cited:
- WO-A-97/43642
- BABA T ET AL: "Preparation and application of a pentamannosyl monophosphate-bovine serum albumin conjugate." CARBOHYDRATE RESEARCH 15 JUN 1988, vol. 177, 15 June 1988 (1988-06-15), pages 163-172, XP002536723 ISSN: 0008-6215
- VALENZANO K J ET AL: "An Assay to Detect Glycoproteins That Contain Mannose 6-Phosphate" ANALYTICAL BIOCHEMISTRY, ACADEMIC PRESS INC, NEW YORK, vol. 209, no. 1, 15 February 1993 (1993-02-15), pages 156-162, XP024763734 ISSN: 0003-2697 [retrieved on 1993-02-15]

### SEQUENCE LISTING

<110> Univeritatsklinikum Hamburg Eppendorf Forschungszentrum Borstel
<120> Antibodies binding mannose 6-phosphate
<130> P 77448
<160> 12
<170> PatentIn version 3.4
<210> 1
   <211> 12
   <212> PRT
   <213> Oryctolagus cuniculus
<400> 1
   Gln Ser Ser Gln Ser Val Tyr Asn Asn Arg Leu Ala
   1               5                   10
<210> 2
   <211> 7
   <212> PRT
   <213> Oryctolagus cuniculus
<400> 2
   Ser Ala Ser Thr Leu Ala Ser
   1               5
<210> 3
   <211> 12
   <212> PRT
   <213> Oryctolagus cuniculus
<400> 3
   His Gly Gly Tyr Arg Ser Asn Asp Asp Arg Tyr Ala
   1               5                   10
<210> 4
   <211> 5
   <212> PRT
   <213> Oryctolagus cuniculus
<400> 4
   Trp Tyr Thr Met Asn
   1 5
<210> 5
   <211> 17
   <212> PRT
   <213> Oryctolagus cuniculus
<400> 5
   Cys Ile Gly Ala Gly Val Tyr Gly Ser Asn Tyr Tyr Ala Ser Trp Ala
   1 5 10 15
   Lys
<210> 6
   <211> 9
   <212> PRT
   <213> Oryctolagus cuniculus
<400> 6
   Asp Gly Ile Asn Gly Gly Tyr Asp Ile
   1 5
<210> 7
   <211> 111
   <212> PRT
   <213> Oryctolagus cuniculus
<400> 7
   Glu Leu Val Met Thr Gln Thr Glu Ser Pro Val Ser Ala Ala Val Gly
   1 5 10 15
   Gly Thr Val Thr Ile Lys Cys Gln Ser Ser Gln Ser Val Tyr Asn Asn
   20 25 30
   Arg Leu Ala Trp Tyr Gln Gln Lys Pro Gly Gln Arg Pro Lys Leu Leu
   35 40 45
   Ile Tyr Ser Ala Ser Thr Leu Ala Ser Gly Val Pro Ser Arg Phe Lys
   50 55 60
   Gly Ser Gly Ser Gly Thr Gln Phe Thr Leu Thr Ile Ser Asp Leu Glu
   65 70 75 80
   Trp Gly Asp Ala Ala Thr Tyr Tyr Cys His Gly Gly Tyr Arg Ser Asn
   85 90 95
   Asp Asp Arg Tyr Ala Phe Ser Gly Gly Thr Glu Leu Glu Ile Leu
   100 105 110
<210> 8
   <211> 117
   <212> PRT
   <213> Oryctolagus cuniculus
<400> 8
   Gln Ser Val Lys Glu Ser Glu Gly Asp Leu Val Lys Pro Gly Ala Ser
   1               5                   10                  15
   Leu Thr Leu Thr Cys Lys Ala Ser Gly Phe Asp Phe Thr Trp Tyr Thr
               20                  25                  30
   Met Asn Trp Val Arg Gln Ala Pro Gly Lys Gly Leu Glu Trp Ile Ala
           35                  40                  45
   Cys Ile Gly Ala Gly Val Tyr Gly Ser Asn Tyr Tyr Ala Ser Trp Ala
       50                  55                  60
   Lys Gly Arg Phe Thr Ile Ser Lys Ala Ser Ser Thr Thr Val Thr Leu
   65                  70                  75                  80
   Gln Met Thr Ser Leu Thr Val Ala Asp Thr Ala Thr Tyr Phe Cys Ala
                   85                  90                  95
   Arg Asp Gly Ile Asn Gly Gly Tyr Asp Ile Trp Gly Pro Gly Thr Leu
               100                 105                 110
   Val Thr Val Ser Ser
           115
<210> 9
   <211> 870
   <212> DNA
   <213> artificial sequence
<220>
   <223> antibody construct
<220>
   <221> CDS
   <222> (1)..(870)
<400> 9
   atg aaa aag aca gct atc gcg att gca gtg gca ctg gct ggt ttc gct       48
   Met Lys Lys Thr Ala Ile Ala Ile Ala Val Ala Leu Ala Gly Phe Ala
   1               5                   10                  15
   acc gtg gcc cag gcg gcc gag ctc gtg atg acc cag act gaa tcg ccc       96
   Thr Val Ala Gln Ala Ala Glu Leu Val Met Thr Gln Thr Glu Ser Pro
               20                  25                  30
   gtg tct gca gct gtg gga ggc aca gtt acc atc aag tgc cag tcc agt      144
   Val Ser Ala Ala Val Gly Gly Thr Val Thr Ile Lys Cys Gln Ser Ser
           35                  40                  45
   cag agt gtt tat aac aac cgt tta gcc tgg tat cag cag aaa cca ggg      192
   Gln Ser Val Tyr Asn Asn Arg Leu Ala Trp Tyr Gln Gln Lys Pro Gly
       50                  55                  60
   caa cgt ccc aag ctc ctg atc tat tct gca tcc act ctg gca tct ggg      240
   Gln Arg Pro Lys Leu Leu Ile Tyr Ser Ala Ser Thr Leu Ala Ser Gly
   65                  70                  75                  80
   gtc cca tcg cgg ttc aaa ggc agt gga tct ggg aca cag ttc act ctc      288
   Val Pro Ser Arg Phe Lys Gly Ser Gly Ser Gly Thr Gln Phe Thr Leu
                   85                  90                  95
   acc atc agc gac ctg gag tgg ggc gat gct gcc act tac tac tgt cat      336
   Thr Ile Ser Asp Leu Glu Trp Gly Asp Ala Ala Thr Tyr Tyr Cys His
               100                 105                 110
   ggc ggt tac cgt agt aat gat gat aga tat gct ttc agc gga ggg acc      384
   Gly Gly Tyr Arg Ser Asn Asp Asp Arg Tyr Ala Phe Ser Gly Gly Thr
           115                 120                 125
   gag ctg gag atc cta tcc tct ggt ggc ggt ggc tcg ggc ggt ggt gga      432
   Glu Leu Glu Ile Leu Ser Ser Gly Gly Gly Gly Ser Gly Gly Gly Gly
       130                 135                 140
   ggt ggt tcc tct aga tct tcc cag tcg gtg aag gag tcc gag gga gac      480
   Gly Gly Ser Ser Arg Ser Ser Gln Ser Val Lys Glu Ser Glu Gly Asp
   145                 150                 155                 160
   ctg gtc aag cct ggg gca tcc ctg aca ctc acc tgc aaa gcc tct gga      528
   Leu Val Lys Pro Gly Ala Ser Leu Thr Leu Thr Cys Lys Ala Ser Gly
                   165                 170                 175
   ttt gac ttc act tgg tat aca atg aat tgg gtc cgc cag gct cca ggg      576
   Phe Asp Phe Thr Trp Tyr Thr Met Asn Trp Val Arg Gln Ala Pro Gly
               180                 185                 190
   aag ggg ctg gag tgg atc gca tgc att ggt gct ggt gtt tat ggt agc      624
   Lys Gly Leu Glu Trp Ile Ala Cys Ile Gly Ala Gly Val Tyr Gly Ser
           195                 200                 205
   aat tac tac gcg agc tgg gcg aaa ggc cgg ttc acc atc tcc aaa gcc      672
   Asn Tyr Tyr Ala Ser Trp Ala Lys Gly Arg Phe Thr Ile Ser Lys Ala
       210                 215                 220
   tcg tcg acc acg gtg acc ctg caa atg acc agt ctg aca gtc gcg gac      720
   Ser Ser Thr Thr Val Thr Leu Gln Met Thr Ser Leu Thr Val Ala Asp
   225                 230                 235                 240
   acg gcc acc tat ttc tgt gcg aga gat ggt att aat ggt ggt tat gac      768
   Thr Ala Thr Tyr Phe Cys Ala Arg Asp Gly Ile Asn Gly Gly Tyr Asp
                   245                 250                 255
   atc tgg ggc cca ggc acc ctg gtc acc gtc tcc tca aga tct gaa cag      816
   Ile Trp Gly Pro Gly Thr Leu Val Thr Val Ser Ser Arg Ser Glu Gln
                   260                 265                 270
   aag cta atc agt gag gaa gat ttg aag gga tcc cat cac cat cac cat      864
   Lys Leu Ile Ser Glu Glu Asp Leu Lys Gly Ser His His His His His
           275                 280                 285
   tag tga                                                              870
<210> 10
   <211> 288
   <212> PRT
   <213> artificial sequence
<220>
   <223> Synthetic Construct
<400> 10
   Met Lys Lys Thr Ala Ile Ala Ile Ala Val Ala Leu Ala Gly Phe Ala
   1               5                   10                  15
   Thr Val Ala Gln Ala Ala Glu Leu Val Met Thr Gln Thr Glu Ser Pro
               20                  25                  30
   Val Ser Ala Ala Val Gly Gly Thr Val Thr Ile Lys Cys Gln Ser Ser
           35                  40                  45
   Gln Ser Val Tyr Asn Asn Arg Leu Ala Trp Tyr Gln Gln Lys Pro Gly
       50                  55                  60
   Gln Arg Pro Lys Leu Leu Ile Tyr Ser Ala Ser Thr Leu Ala Ser Gly
   65                  70                  75                  80
   Val Pro Ser Arg Phe Lys Gly Ser Gly Ser Gly Thr Gln Phe Thr Leu
                   85                  90                  95
   Thr Ile Ser Asp Leu Glu Trp Gly Asp Ala Ala Thr Tyr Tyr Cys His
               100                 105                 110
   Gly Gly Tyr Arg Ser Asn Asp Asp Arg Tyr Ala Phe Ser Gly Gly Thr
           115                 120                 125
   Glu Leu Glu Ile Leu Ser Ser Gly Gly Gly Gly Ser Gly Gly Gly Gly
       130                 135                 140
   Gly Gly Ser Ser Arg Ser Ser Gln Ser Val Lys Glu Ser Glu Gly Asp
   145                 150                 155                 160
   Leu Val Lys Pro Gly Ala Ser Leu Thr Leu Thr Cys Lys Ala Ser Gly
                   165                 170                 175
   Phe Asp Phe Thr Trp Tyr Thr Met Asn Trp Val Arg Gln Ala Pro Gly
               180                 185                 190
   Lys Gly Leu Glu Trp Ile Ala Cys Ile Gly Ala Gly Val Tyr Gly Ser
               195                 200                 205
   Asn Tyr Tyr Ala Ser Trp Ala Lys Gly Arg Phe Thr Ile Ser Lys Ala
       210                 215                 220
   Ser Ser Thr Thr Val Thr Leu Gln Met Thr Ser Leu Thr Val Ala Asp
   225                 230                 235                 240
   Thr Ala Thr Tyr Phe Cys Ala Arg Asp Gly Ile Asn Gly Gly Tyr Asp
                   245                 250                 255
   Ile Trp Gly Pro Gly Thr Leu Val Thr Val Ser Ser Arg Ser Glu Gln
               260                 265                 270
   Lys Leu Ile Ser Glu Glu Asp Leu Lys Gly Ser His His His His His
                   275                 280                 285
<210> 11
   <211> 37
   <212> DNA
   <213> Artificial
<220>
   <223> PCR oligonucleotide primer
<400> 11
   ggggggaatt catgaaaaag acagctatcg cgattgc 37
<210> 12
   <211> 69
   <212> DNA
   <213> Artificial
<220>
   <223> PCR oligonucleotide primer
<400> 12
   gggggatccc ttcaaatctt cctcactgat tagcttctgt tcagatcttg aggagacggt     60
   gaccagggt                                                             69

## Description

The present invention relates to an isolated antibody or antigen-binding fragment thereof which specifically binds to mannose 6-phosphate (Man6P) and methods for preparing such antibody or antigen-binding fragment thereof. The invention further relates to a nucleic acid molecule encoding such antibody or antibody fragment, an expression vector comprising such nucleic acid molecule and a host cell comprising such nucleic acid molecule or expression vector. The invention also relates to a method for purifying or concentrating a target molecule comprising at least one Man6P residue from a sample. In another aspect, the invention relates to a method for diagnosing a disease which is characterized by an absent or reduced modification of molecules with mannose-6-phosphate residues. Finally, the invention relates to the use of the isolated antibody or antibody fragment of the invention for use in medicine, in particular for the diagnosis of a disease which is characterized by an absent or reduced modification of molecules with mannose-6-phosphate residues.

### BACKGROUND OF THE INVENTION

Lysosomes are cell organelles surrounded by membranes which function in the intracellular enzymatic degradation of macromolecules, such as proteins, carbohydrates, lipids and nucleic acids. Lysosomes therefore contain a large number of hydrolases, such as proteases, nucleases and lipases. The macromolecules to be degraded are transported to lysosomes either by endocytosis from the extracellular space or by fusion with autophagosomes. In lysosomes, macromolecules are enzymatically digested into their components which are subsequently transported across the lysosomal membrane into the cytosol. For facilitating macromolecule degradation, lysosomal hydrolases require an acidic environment which is established by ATP-driven proton pumps located in the membrane of the lysosome. The interior of the lysosome has a pH of 4.5 to 5.0, while the cytosol of the cell has a pH of about 7.2.

For the function and integrity of existing lysosomes, it is necessary that both lysosomal enzymes and membrane proteins are continuously replaced. Lysosomal hydrolases are synthesized and glycosylated in the rough endoplasmatic reticulum, transported to the Golgi apparatus where they are sorted and subsequently transported to the lysosomes. This protein sorting occurs by means of a specific oligosaccharide marker mediating the efficient transport to the lysosomes. Oligosaccharides of newly synthesized lysosomal enzymes are specifically modified in the Golgi apparatus with Man6P residues which function as recognition marker for Man6P receptors present in the trans-Golgi network. Ligand-receptor-complexes are then packed into clathrin-coated vesicles which are transported to and fused with endosomes. A pH-induced dissociation of the receptor-ligand complexes releases the enzymes which are then delivered to lysosomes. The initial step in the formation of Man6P residues is catalysed by two different Golgi-resident enzymes. In a first step, the enzyme GlcNAc-1-phosphotransferase catalyses the formation of a covalent phosphodiester bond between the 6-OH group of an α1.2-linked mannose moiety and α-GlcNAc 1-phosphate using uridine diphosphate GlcNAc as substrate. In a second step, the GlcNAc phosphodiester is cleaved by the catalytic activity of a phosphodiesterase releasing GlcNAc and exposing the Man6P marker.

Defects in the enzyme GlcNAc-1-phosphotransferase which result in a significant reduction or even in a complete block of the Man6P recognition signal synthesis are known to be responsible for two inherited rare disorders which are characterized by missorting of lysosomal enzymes and lysosomal accumulation of non-degraded macromolecules. In patients suffering from mucolipidosis type II (ML-II) or type III (ML-III), lysosomal enzymes are synthesized normally in the endoplasmatic reticulum but their insufficient or missing Man6P label prevents recognition by Man6P receptors. Consequently, the enzymes are not transported to lysosomes, but instead secreted into the extracellular space. ML-II, also known as I-cell-disease, is regularly associated with severe physical and developmental delays in the affected individual. Symptoms of ML-II typically occur at birth or shortly thereafter and may include short stature, stiff hands, kyphosis (curvature of the spine), genu valgum (knock-knees), coarse facial features, gingival hyperplasia (overgrowth of the gums), corneal clouding, organomegaly (enlarged organs), hernias, repeated respiratory infections, heart problems and severe motor and mental retardation. The lifespan of individuals affected with ML-II is typically less than 10 years.

ML-III, also known as pseudo-Hurler polydystrophy, is a milder form of the disease. Symptoms of ML-III are usually noted in individuals between 2 and 4 years of age and may include short stature, stiff joints, coarse facial features, problems with the valves of the heart and corneal clouding. Half of the affected individuals have learning disabilities. Many of the symptoms of ML-II can be present in ML-III, but are usually less severe allowing the survival into the eighth decade.

The diagnosis of ML-II or ML-III is based on the assessment of the clinical symptoms of the disease and enzyme activity assays. In particular, increased serum levels or the hypersecretion of lysosomal enzymes accompanied by an intracellular deficiency of multiple lysosomal enzymes detected in cultured cells of patients are used as diagnostic markers. However, there is a high variability in the normal range of lysosomal enzyme activities depending on the source of tested samples (e.g. dry blot spots, lymphoblasts, cultured skin fibroblasts or amniotic cells), the nature of mutations in the GlcNAc-1-phosphotransferase gene, and the substrates used in activity assays. Consequently, the results obtained in these assays may vary considerably making a reliable diagnosis of ML-II or ML-III complicated and additional labor intensive molecular analysis by sequencing of several genes necessary.

Accordingly, it was an objective of the invention to provide means and methods which allow for the reliable diagnosis of diseases which are characterized by an absent or reduced synthesis of the Man6P signal on molecules, such as lysosomal polypeptides. This objective is achieved by the provision of an antibody as defined in the attached claims.

### BRIEF DESCRIPTION OF THE FIGURES

Fig. la shows the ligand binding specificity of the single-chain antibody fragment scFv M6P-1.
Fig. 1b shows the results from immunoprecipitation of [¹²⁵I]-labeled lysosomal arylsulfatase A with scFv M6P-1-coupled beads in the absence (-) and presence of increasing concentrations of mannose 6-phosphate (Man6P). The concentrations of Man6P are indicated in mM.
Fig. 1c depicts the ITC microcalorimetry results using a 0.12 mM solution of scFv M6P-1 in PBS. The results of 20 injections (4 µL each) of 10 mM Man6P are shown.
Fig. 2a shows that scFv M6P-1 serves as a tool for the diagnosis of mucolipidosis II. Extracts of cultured fibroblast cells (C) and conditioned media (M) of both healthy control individuals or of ML-II patients were analysed by Western blotting using scFv M6P-1. The positions and molecular masses in kDa of marker proteins are indicated.
Fig. 2b shows that scFv M6P-1 serves as a tool for the diagnosis of mucolipidosis III. Extracts of cultured fibroblast cells and conditioned media of both healthy control individuals or of three ML-III patients were analysed by Western blotting using scFv M6P-1. The positions and molecular masses in kDa of marker proteins are indicated.
Fig. 3 shows the purification of Man6P-containing lysosomal enzymes from media of BHK cells which express these enzymes using scFv M6P-1 coupled to beads.
Fig. 4 shows the nucleotide sequence and the deduced amino acid sequence of the scFv M6P-1 expression construct.

### DETAILED DESCRIPTION OF THE INVENTION

According to the present invention, an isolated antibody fragment is provided which specifically binds mannose 6-phosphate (Man6P) as defined in the claims. Because Man6P is a characteristic structural component of lysosomal polypeptides the present invention also provides a method for the rapid and efficient diagnosis of diseases which are characterized by an impaired or failed formation of the Man6P marker on lysosomal enzymes.

In particular, the present invention discloses the generation of a recombinant single-chain antibody fragment which specifically binds to Man6P. The antibody fragment was obtained by immunisation of rabbits with a neoglycoprotein of the pentamannose 6-phosphate (PMP) fraction of the yeast *Hansenula holstii* that was conjugated to, bovine serum albumin (PMP-BSA). The PMP of *Hansenula holstii* is known to contain terminal Man6P residues (Parolis, L.A., et al. (1998), Carbohydr. Res. 309, 77-87; Parolis, L.A., et al. (1996), Carbohydr. Res. 293, 101-117). RNA isolated from bone marrow cells of immunised rabbits was used for the generation of a library of phage-displayed scFv antibody fragments as described in the examples. Antibody fragments with binding affinity to Man6P were isolated by panning techniques, and a single scFv fragment (designated scFv M6P-1) which specifically binds to PMP was isolated and characterized. The identification of the complementarity-determining regions (CDRs) of the isolated fragment facilitates the preparation of recombinant antibodies which exhibit specificity for Man6P and may be used in diagnosis and as a research tool for the study of mechanisms underlying polypeptide targeting to lysosomes. Knowledge on the CDRs of an antibody specific for Man6P may also be useful in techniques like CDR-grafting to prepare full-length antibodies of different classes or fragments thereof having the desired binding specificity to Man6P.

The present invention therefore relates to an isolated antibody or an antigen-binding fragment thereof which specifically binds to Man6P. In the context of the present invention, the term "antibody" includes monoclonal antibodies, polyclonal antibodies, multispecific antibodies (for example, bispecific antibodies), anti-idiotypic antibodies, chimeric antibodies, and humanized antibodies, as long as they exhibit the desired immunological activity, i.e. specific binding to Man6P. In particular, the term is to be understood as comprising any kind of antibody molecule that was prepared by grafting one or more of the CDRs of the single-chain antibody fragment disclosed herein. As used herein, the term comprises antibody molecules of any isotype class, such as IgG, IgM, IgA, IgE, and IgD as well as their subclasses, e.g., IgG1, IgG2 and the like. The term also comprises uncommon antibodies, such as IgY from chicken, IgW from sharks and single domain antibodies from camels and llamas. As used herein, an antibody comprises at least two identical heavy (H) chains and two identical light (L) chains. These polypeptide chains are joined together by disulfide bonds. The heavy chain of an antibody consists of a heavy chain variable region (V_{H}) and a heavy chain constant region, the latter of which is comprised of three domains referred to as C_{H}1, C_{H}2 and C_{H}3. In some cases, it may also comprise more than three C_{H} domains (for example, human IgE or IgNARC of cartilaginous fish).

The light chain consists of a light chain variable region (V_{L}) and a light chain constant region (C_{L}). In the native antibody, the C_{L} region of the light chain is aligned with the C_{H}1 region, i.e., the most N-terminal constant domain of the heavy chain. Similarly, the V_{L} region is aligned in the native antibody with the heavy chain V_{H} region.

The V_{H} and V_{L} regions of the heavy and the light chain contain three amino acid segments referred to "complementarity-determining regions" (CDRs). These segments within the variable region are responsible for binding an epitope on the antigen to which the antibody is directed. The CDRs are numbered sequentially starting from the N-terminus of the polypeptide chain (CDR1, CDR2, and CDR3). The CDRs are interrupted by conserved regions that are designated framework regions. Each V_{H} and V_{L} region starts with a framework region at its N-terminus. Accordingly, each V_{H} and V_{L} region consists of three CDRs and four framework regions. The person of skill is readily able to determine CDRs and framework regions in a given polypeptide sequence, for example, in a variable region of a heavy or light chain of an antibody. For example, CDRs and framework regions may be identified by comparative analysis of a sequence in question with known antibody primary structures. Reference is also made to the methods described in MacCallum, R.M. et al. (1996) J. Mol. Biol. 262, 732-745, which describe the formation of crystallized antibodies in complex with antigen resulting in the identification of key amino acid residues which are often involved in antigen contact.

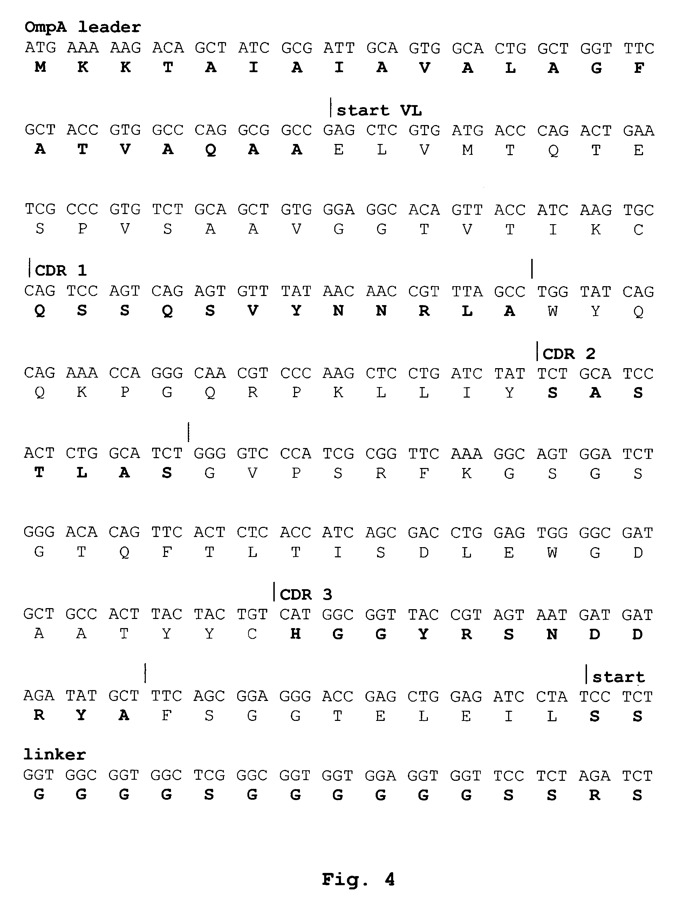

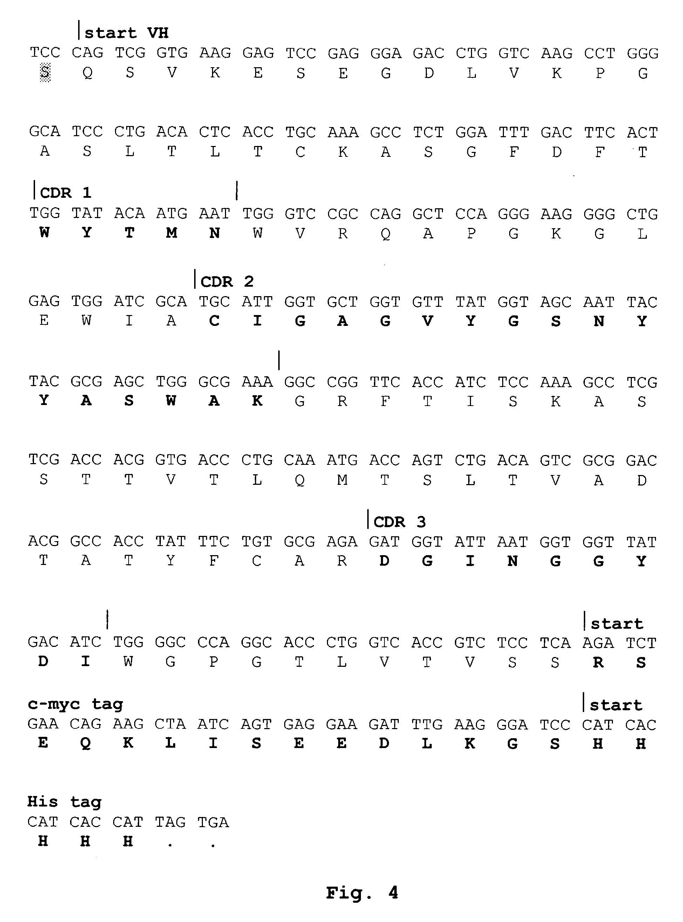

The CDRs of the V_{H} and V_{L} region of the antibody fragment identified in the present invention are depicted in Fig. 4 and SEQ ID NOs: 1-6. The amino acid sequences of the CDRs of the V_{L} region are provided in SEQ ID NOs: 1-3, and the CDRs of the V_{H} region are provided in SEQ ID NOs: 4-6. According to a preferred aspect, the invention refers to an isolated antibody or an isolated antigen-binding fragment thereof which specifically binds to Man6P and which comprises the CDRs shown in SEQ ID NOs:1-6 or homologs thereof.

Generally, the CDRs of the V_{H} and V_{L} domains of antibodies are involved in the formation of the antigen binding site by creating a surface complementary to the antigen. Thus, an antibody fragment which contains a complete antigen-binding site comprises the CDRs of the V_{H} and V_{L} region of the whole antibody. These regions interact with each other, so that their CDRs define the structure of the antigen-binding site. Usually, the interaction with the antigen involves contacts provided by side chains or backbone atoms of amino acids forming the antibody CDRs. Thus, not all CDRs may be required for antigen binding and it has been recognized that the CDR3 of the V_{H} often is particularly important for antigen recognition. It is also contemplated by the invention to use antibody fragments that comprise either all CDRs of the V_{H} or V_{L} regions, selected CDRs of these in combination with different CDRs not relevant for antigen binding or portions of the CDRs retaining stretches of amino acids relevant for antigen binding. It has been found that fragments which only comprise the CDRs of the V_{H} or V_{L} region, respectively, may also be capable of recognizing and binding to the antigen.

In a preferred embodiment of the invention, the antibody or antigen-binding fragment comprises at least a portion of a light chain variable region, said portion containing the CDRs shown in SEQ ID NOs:1 and 2 or homologs thereof In a further preferred embodiment of the invention, the antibody or antigen-binding fragment comprises at least a portion of a light chain variable region, said portion containing the CDRs shown in SEQ ID NOs:1-3 or homologs thereof and at least a portion of a heavy chain variable region, said portion containing the CDRs shown in SEQ ID NOs:4-6 or homologs thereof. In other words, an antibody or an antigen-binding fragment comprising all 3 CDRs of the V_{H} region and all 3 CDRs of the V_{L} region is contemplated. According to a more preferred embodiment, the antibody or antigen-binding fragment comprises both at least a portion of a light chain variable region, wherein said portion contains the CDRs shown in SEQ ID NOs:1-3 (or homologs thereof) and a portion of a heavy chain variable region, wherein said portion contains the CDRs shown in SEQ ID NOs:4-6 (or homologs thereof). It is particularly preferred that the antibody or antigen-binding fragment of the invention comprises the light chain variable region and/or the heavy chain variable region of the antibody fragment scFv M6P-1 disclosed herein, i.e. the light chain variable region shown in SEQ ID NO:7 and/or the heavy chain variable region shown in SEQ ID NO:8. The invention also comprises embodiments where the antibody or antigen-binding fragment of the invention comprises a light chain variable region which is homologous to that shown in SEQ ID NO:7 and/or a heavy chain variable region which is homologous to that shown in SEQ ID NO:8. In one aspect, the antibody or antigen-binding fragment comprises the light chain variable region of scFv M6P-1 (SEQ ID NO:7) and the heavy chain variable region (SEQ ID NO:8) of scFv M6P-1 or regions which are homologous thereto, respectively.

Apart from whole antibodies, the present invention also refers to antigen-binding fragments of an antibody which specifically bind to Man6P. As used herein, an antigen-binding fragment of an antibody according to the invention is a fragment which retains the specific binding activity for Man6P. Antigen-binding fragments of the invention may comprise Fv, Fab, F(ab') and F(ab')₂ fragments. Further included by the term "antigen-binding fragment" are single chain Fv antibody fragments (which are also designated "scFv antibodies herein) and disulfide-linked Fv fragments (dsFv). Single chain antibody fragments (scFv) comprise the variable domain of the heavy chain (V_{H}) and variable region of the light chain (V_{L}) of an antibody, wherein these regions are linked by a peptide bridge or by disulfide bond. Preferably, the V_{H} region and the V_{L} region are linked by a flexible amino acid linker which enables the regions to interact with each other to form the antigen-binding site. According to a particularly preferred embodiment of the present invention, the antibody fragment which specifically binds to a Man6P residue is a single-chain variable antibody fragment (scFv), for example, the single-chain variable antibody fragment depicted in Fig.4. Methods for recombinantly producing scFv antibody fragments have been described in the prior art and comprise, for example, techniques such as phage display or ribosome display. The recombinant scFv antibody fragments produced by these methods may be purified and tested for binding affinity to proteins having one or more Man6P residues, for example, lysosomal proteins.

The antibody of the present invention and its antigen-binding fragments specifically bind to Man6P. This means that the antibody or antigen-binding fragment thereof will not exhibit any biological significant binding to molecules other than Man6P or molecules containing Man6P residues (such as lysosomal enzymes). As used herein, the term is used in a way which does not exclude the binding of the antibody and its antigen-binding fragments to fructose-1-phosphate (Fru1P). It was previously reported that Fru1P is able to compete with the binding of lysosomal enzymes to Man6P receptors (Olson, L. et al., J Biol Chem (1999), 274(42):29889-96; von Figura K and Weber E, Biochem J (1978), 176:943-950), and it can also compete with the binding of antibody fragment scFv M6P-1 to PMP. The similar properties of Fru1P and Man6P as inhibitors can be explained by structural similarities. The capability of the binding of Fru1P is, however, not biologically significant, and it does not interfere with the methods disclosed in the present invention. This is because Fru1P is a cytosolic component present in cells in a very low concentration that does not occur as posttranslational modification of molecules such as lysosomal enzymes (Davies, D.R., et al. (1990) Eur. J. Biochem. 192:283-289).

As used in the context with an antibody or an antibody fragment, "isolated" means that the antibody or antibody fragment is present in a form other than in its native environment. This means that the isolated antibody or an isolated antibody fragment is substantially free of contaminants such as other antibodies that regularly occur, for example, in the blood of an immunised animal. For example, polyclonal antibodies are not "isolated" antibodies in the sense of the present invention. As used herein, the term does however not exclude the presence of the same antibody or antibody fragment in alternative forms, such as in the form of a multimer, for example a dimer, or in an alternatively glycosylated form.

An antibody or antibody fragment which specifically binds to Man6P may be produced by a number of different methods known in the art. For example, an antibody to a given antigen may be generated by immunisation of an animal with the antigen, as it is described in the examples. Alternatively, given the sequence information provided herein, it is readily possible to generate an antibody based on recombinant methods, such as PCR techniques. The antibody or the antigen-binding fragment thereof may be (or may be derived from) a monoclonal antibody. Methods for generating monoclonal antibodies are well-known in the art and described in several standard textbooks (e.g. in Harlow and Lane, Antibodies: A Laboratory Manual, Cold Spring Harbor Laboratory Press (1998)).

As used herein, a "monoclonal antibody" refers to an antibody produced by a cell line that is based on a single B lymphocyte. Monoclonal antibodies are directed against a single epitope in the particular antigen to which the antibody was raised. Thus, the CDRs of a monoclonal antibody are identical in all molecules of a population of a monoclonal antibody. A monoclonal antibody may be produced by a method which provides for the production of antibody molecules in continuous cell lines. For example, monoclonal antibodies can be produced by hybridoma cell lines as described by Köhler and Milstein, Nature, 256, 495-397, (1975); and Ausubel et al., (eds.), 1998, Current Protocols in Molecular Biology, John Wiley & Sons, New York. The method described by these authors is based on fusion of an antibody-producing B cell with an immortalized cell line resulting in a hybrid cell that produces antibodies of a defined specificity to an unlimited extent. In a first step, the antigen, for example, the conjugate consisting of bovine serum albumin and pentamannose 6-phosphate (PMP-BSA) described in Example 1, is injected into an animal, preferably into a mammal such as a mouse, hamster, goat, rabbit, etc., thereby stimulating the production of antibodies against the antigen in the B lymphocytes. Spleens from the immunised animals are collected and processed to obtain a cell suspension for use in fusion with an immortalized cell line. The immortalized cell lines which may be used in this context are in particular transformed mammalian cells, such as myeloma cells of murine or human origin. The hybridoma cells resulting from the fusion are cultured in an appropriate selective culture medium, and culture medium from each hybridoma is assayed for monoclonal antibodies which are directed against the antigen. Once hybridoma cells which produce the desired monoclonal antibody have been identified, DNA from these clones may be cloned by methods known in the art for expression by recombinant methods. Several other methods have been described in the art for the production of monoclonal antibodies, for example, methods which are based on antibody phage libraries.

In another aspect of the invention, the antibody or antibody fragment of the invention is a recombinant antibody or a recombinant antigen-binding fragment. As used herein, the term "recombinant" in the context with an antibody or antibody fragment means that the antibody or antibody fragment has not been derived directly from a B lymphocyte of an immunised animal, but rather results from human intervention based on recombinant DNA techniques. For example, as described in the examples provided herein, antibody fragments such as scFv fragments may be obtained by using phage display methods which result in the random combination of V_{H} and V_{L} regions. The term also comprises any kind of antibody or antibody fragment which results from grafting parts of an antibody obtained from a B lymphocyte (for example CDRs) to another antibody molecule. Preferred examples of recombinant antibody fragments according to the invention are single-chain variable antibody fragments (scFv).

It will be appreciated that the antibodies or antibody fragments of the invention are not restricted to those comprising the CDR sequences specifically exemplified in SEQ ID NOs:1-6, but also encompass antibodies or antibody fragments comprising CDR sequences that are homologous to the specifically exemplified CDRs in SEQ ID NOs:1-6. As used herein, the term "homologous" or "homolog" refer to an amino acid sequence which has been modified by deletion, substitution or addition of amino acids to show at least 80%, 85%, 90%, 91%, 92%, 93%, 94%, 95%, 96%, 97%, 98% or 99% sequence homology to another amino acid sequence if these sequences are optimally aligned, for example by the programs GAP or BESTFIT using default gap. As used herein, the term "homologous" or "homolog" also comprises cases where an amino acid sequence shows at least 75%, 80%, 85%, 90%, 91%, 92%, 93%, 94%, 95%, 96%, 97%, 98%, or 99% sequence identity to another amino acid sequence in an optimal alignment. For example, a sequence identity of 90% means that 90% of the amino acids of an analyzed amino acid sequence stretch are identical to the sequence of the reference amino acid sequence. Methods and computer programs for determining amino acid sequence homologies are well known in the art.

The CDR sequences may differ from those depicted in SEQ ID NOs:1-6 by the replacement of 1, 2 or 3 amino acids. Generally, each of the amino acid residues within the CDRs of SEQ ID NOs:1-6 may be substituted by another residue, as long as the resulting CDR is still capable (in concert with the other CDRs) of specifically binding Man6P. It is preferred that the substitutions are conservative substitutions, i.e. substitutions of one or more amino acid residues by an amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

According to a further aspect, the invention relates to a nucleic acid molecule encoding an antibody or an antigen-binding fragment of the invention. The invention also relates to an expression vector which comprises a nucleic acid molecule encoding an antibody or an antigen-binding fragment of the invention. An expression vector will comprise the nucleic acid molecule encoding an antibody or an antigen-binding fragment operatively linked to control sequences which effect expression of the nucleic acid molecule, for example, promotor sequences, enhancer sequences, and the like.

In another aspect, the present invention relates to a host cell comprising a nucleic acid molecule or an expression vector as described above. The host cell is preferably a non-human cell and may be a prokaryotic or eukaryotic host cell. Examples for host cells which can be used according to the invention comprise prokaryotic host cells such as *E*. *coli*, *Bacillus subtilis,* and the like. Suitable eukaryotic cells comprise yeast cells, insect cells, mammalian cells, plant cells and the like. Preferably, the antibody or antigen-binding fragment of the invention is expressed in bacterial cells such as *E*. *coli.* The method to be used for transforming or transfecting the host cell with an expression vector comprising the nucleic acid molecule encoding the antibody or antigen-binding fragment of the invention will depend both on the type of expression vector and the host cell used. The skilled person will have no problems to choose appropriate vectors and host cells for recombinant expression. The invention also provides a method for preparing an antibody or antibody fragment of the invention, wherein any of the host cells described above is cultured under conditions which allow for the expression of the nucleic acid molecule encoding the antibody or the antigen-binding fragment of the invention.

According to a further aspect, the present invention relates to a method for purifying or concentrating a target molecule which comprises at least one Man6P residue from a sample, using an antibody or antibody fragment of the invention. According to a preferred embodiment, the target molecule to be purified or concentrated is a polypeptide, for example, a lysosomal enzyme. In the context of the present invention, the term "purifying" means that the target molecule is essentially separated from other compounds which are present in the sample. It will be recognized that the term does not exclude the possibility that certain impurities (other compounds than the target molecules) may be present in the sample. Preferably, a purified target molecule is more than 90% pure, more preferably more than 95% pure and even more preferably more than 98% pure. According to a particular preferred embodiment, the target molecule is more than 99% pure. The term "concentrating" refers to the increase of the portion of a target molecule in a sample relative to the content of other compounds in the sample, or relative to the content of said target molecule in a non-concentrated sample.

The target molecule to be purified or concentrated according to the invention is characterized by the content of one or more Man6P residues. The target molecule can be a molecule that comprises one or more Man6P residues in its naturally occurring form. For example, the target molecule can be a lysosomal enzyme that is synthesized and subsequently modified in a mammalian cell with one or more Man6P residues for the targeted transport of the enzyme to lysosomes. Alternatively, the target molecule can be a molecule which does not contain a Man6P residue in its naturally occurring form.

According to a particularly preferred embodiment of the invention, the target molecule which is to be purified or enriched according to the invention is a molecule contemplated in lysosomal enzyme replacement therapy (ERT). In this kind of therapy, a lysosomal enzyme lacking in an individual to be treated is prepared by recombinant methods, purified and subsequently administered into the blood stream of said individual. The respective recombinant enzyme is internalized by the cells via receptor-mediated endocytosis and transported to lysosomes allowing the degradation of disease-causing storage material. Pharmaceutical products for use in ERT are currently marketed for M. Gaucher, M. Fabry, mucopolysaccharidosis type I (M. Hurler; M. Scheie), type II (M. Hunter), type VI (M. Maro-teaux-Lamy), and M. Pompe, and clinical trials are underway for several other diseases. The enzymes used in replacement treatment are regularly produced in expression systems which guarantee their labeling with Man6P residues. This means that the enzymes have to be produced in mammalian expression systems rather than in bacterial or insect systems. Thus, the enzymes are produced in an established mammalian cell line, for example in CHO-S cells. The cells may be transformed or transfected with an expression vector encoding the lysosomal enzyme of interest. The cells are cultured in suspension in a suitable culture medium under conditions which promote expression and secretion of the enzyme. The recombinant enzyme can be purified from collected media by means of several chromatographic purification steps and/or their affinity to the antibody or antibody fragment of the invention.

The sample containing the target molecule normally also comprises compounds and molecules which are different from the target molecule. In a preferred embodiment, the sample is a culture medium conditioned by cells, i.e. a medium containing a heterogeneous mixture of different secreted macromolecules, such as glycosylated and non-glycosylated polypeptides. As outlined above, the sample may be a medium from cells expressing the recombinant lysosomal enzyme for ERT.

The method comprises a step in which an antibody or an antibody fragment of the invention is incubated with the sample. The incubation step is carried out under conditions which are suitable for the formation of binding complexes consisting of the antibody or antibody fragment on the one hand and the target molecule on the other. Conditions which allow for the formation of binding complexes of the antibody of the invention and its antigen are generally known in the art and will normally include moderate salt and neutral pH conditions, a temperature in the range of 4-37°C, and an incubation period between several minutes and several hours. It will be appreciated by those of ordinary skill in the art that suitable binding conditions for a given antibody-antigen pair will also considerably depend on factors like the nature, affinity and concentration of the antibody or fragment used, the type of sample to which the antibody or fragment is employed, and the like. The skilled person will readily be able to adjust these conditions simply by performing routine experiments to optimize the formation of the binding complexes.

Upon contact to the target molecule under suitable conditions, the antibody or antibody fragment will bind to Man6P residues of the target molecule in the sample, resulting in binding complexes comprising the antibody/antibody fragment and the target molecule presenting the antigenic Man6P structure. The binding complexes are separated from the sample, thereby purifying or concentrating the target molecule. Preferably, the method contemplated for purifying or concentrating the target molecule is a one-step method which means that only a single step is required to obtain the target molecule in a substantially purified or concentrated form.

The antibody or antibody fragment of the invention may be immobilized on a solid support, for example, on a resin that is suitable for being used in chromatographic techniques. The antibody or antibody fragment is preferably bound to a solid support such as sepharose, agarose, or other suitable solid-support medium. In an alternative embodiment, the antibody or antibody fragment of the invention is immobilized to beads, for example, agarose or glass beads. Preferably, the beads are magnetizable, thereby facilitating separation of the binding complexes coupled to the beads from the sample. Methods for coupling an antibody or antibody fragment to a solid support are widely known in the art. For example, one method suitable for coupling makes use of a covalent derivatization with appropriate derivatives of biotin and subsequent capturing onto surfaces, e.g. beads, coated with avidin, streptavidin or derivatives thereof. Such compounds are commercially available from several manufacturers. Alternatively, the protein may be directly conjugated to the solid support by chemical reactions known in the art and according to instructions of commercially available systems.

One method for non-covalent coupling of whole antibodies (including a Fc region) makes use of protein A from *Staphylococcus aureus* which binds to the Fc region of most mammalian IgG antibodies. Protein A-coupled resins are commercially available from several manufacturers. When applying the sample containing the target molecule to the antibody or antibody fragment immobilized on the resin, this will result in a selective absorption of the target molecule to the immobilized antibody or antibody fragment. By use of a suitable washing buffer, any unbound or weakly bound molecules are washed from the resin, while the target molecule is retained. In a final step, the target molecule can be eluted with a suitable elution buffer.

In embodiments where the antibody or antibody fragment of the invention is not immobilized to a support, separation can be affected, for example, by using fusion polypeptides comprising (apart from the antibody or antibody fragment of the invention) an affinity tag which allows the binding of the fusion polypeptide via a compound exhibiting binding affinity to the tag. For example, the affinity tag may be a poly-histidine tag comprising 6-12 histidine residues which specifically interacts with a nickel ion chelate matrix. Alternatively, the tag may be glutathione-S-transferase allowing the purification on a glutathione matrix. Further affinity tags are well-known in the art. Non-limiting examples for pairs of affinity tag and affinity ligand include maltose-binding-protein (MBP) and maltose; avidin and biotin; Streptag and streptavidin or neu-travidin. Affinity tags may be attached to a molecule by fusion/ligation of cDNAs of the molecule of interest with the tag-encoding sequence. In cases where both the tag and the molecule to be modified is a peptide or polypeptide, a fusion polypeptide may be generated comprising the amino acid sequences of the target polypeptide and the affinity tag. In other embodiments, the affinity tag may be attached by chemical coupling reactions. For example, biotin may be chemically coupled to different target molecules. Biotin labeling can also be achieved by attaching a peptide sequence to the protein which is recognized by a biotin protein ligase, e.g. BirA in E. coli, and coexpression of birA then leads to biotinylation of the tag sequence, a conserved Lys-residue (see, for example, Smith P. et al. Nucleic Acids Research (1998) 26:1414-1420; Becket et al. Protein Science (1999) 8: 921-929).

The invention also relates to a method for identifying a target molecule which comprises at least one Man6P residue in a sample, using an antibody or antibody fragment of the invention. The term "identifying" refers to the detection of target molecules among other components that are present in the sample. In a first step, an antibody or antibody fragment as described herein is contacted with said sample under conditions which allow for the formation of binding complexes comprising the antibody or antibody fragment and the target molecule (see above). In a subsequent step, binding complexes which may have formed in the sample are detected, thereby identifying the target molecule.

In a further aspect, the antibody or antigen-binding fragment of the invention is contemplated herein for use in medicine. For example, a method is provided for diagnosing diseases which are characterized by the absent or reduced modification (labeling) of molecules, preferably polypeptides, with Man6P residues. This means that certain molecules of the individual who is afflicted with the disease show a pathological reduction in the extent of Man6P modification or even a complete loss of Man6P modification. Preferably, the disease to be diagnosed according to the invention is characterized by an abnormal Man6P modification of polypeptides, more preferably enzymes such as lysosomal enzymes. Such diseases have been described in the prior art. For example, mucolipidosis type II and type III are diseases in which a defect of the enzyme GlcNAc-1-phosphotransferase causes a reduction or loss in the enzymatic activity of this enzyme. The reduced or absent GlcNAc-1-phosphotransferase activity results in an impaired attachment of the Man6P label to lysosomal enzymes which in turn causes missorting and intracellular depletion of these enzymes.

The diagnostic method of the present invention comprises contacting any of the antibodies or antibody fragments described herein with a cell sample from an individual who is suspected of being affected by said disease. The contacting step is performed under conditions which allow for the formation of binding complexes comprising the antibody or antibody fragment and a molecule comprising at least one Man6P residue. As described above, these conditions can be readily determined by a person of ordinary skill in the art and normally include moderate salt and neutral pH conditions, a temperature in the range of 4-37°C, and incubation times between several minutes and 24 hours.

In a subsequent step, the degree of binding of said antibody or antibody fragment to molecules of the sample comprising at least one Man6P residue is determined. This means that the extent of binding of the antibody or antibody fragment to molecules of the sample is quantitatively assessed, e.g. by detecting the amount of binding complexes that are formed upon incubation of the antibody or antibody fragment with the Man6P-containing antigen in the sample. Methods for the evaluation of antibody binding are known in the art.

Experimental methods suitable for quantifying complexes between ligand and antibody frequently detect the equilibrium at varying concentrations, preferably at 50% saturation, through any signal that is proportional to the degree of saturation. Appropriate signals comprise, e.g. heat, surface plasmon resonance, acoustic waves, intensity of fluorescence and the like. Titration of one binding partner, preferably the smaller antigen, to a solution of antibody or antigen binding fragments will allow the person of skill to determine the concentration of free ligand which is necessary to achieve half-maximal saturation of binding sites on the antibody. From these measurements an equilibrium binding constant K can be derived. Alternatively, binding constants can be derived by mathematical fitting of experimentally determined to theoretical values. Alternatively, the amount of formed complex can be derived from determining the amount of antibody retained on immobilized ligand or vice versa. Since carbohydrates, such as Man6P, do not adhere efficiently to surfaces, e.g. plastic surfaces or filter membranes which are used in such assays, they are frequently tethered chemically to molecules of other kind, e.g. proteins as neoglycoconjugates or polymers, which allows their indirect immobilisation. Quantification of the retained binding partner due to specific complex formation can then be achieved either indirectly or directly, if appropriately, e.g. radio- or fluorescently, labelled. The indirect quantification relies on the use of secondary, labelled antibodies which may be fluorescent but more often have been modified with an enzymatic activity, such as e.g. horseradish peroxidase or alkaline phosphatase (Enzyme-linked immunosorbent assay, ELISA). The amount of retained antibody is then determined from the concentration of an enzymatically formed chromophore measured by the absorbance at a specific wavelength. The complex formation between ligand and antibody can further be measured by biosensor technologies (e.g. surface plasmon resonance, surface acoustic wave) which follow binding events in real time and derive K values either at equilibrium or from rate constants of association and dissociation phases. Moreover, quantification of lysosomal enzymes retained to antibody-coupled beads can be achieved by determining the enzymatic activity for lysosomal enzymes of interest using commercially available substrates (e.g. p-nitrophenyl or methylumbelliferyl substrates) or by quantitative Western blotting with recombinant lysosomal enzymes of interest as standard and specific antibodies against the lysosomal enzyme.

The degree of binding is then compared to the degree of binding of the same antibody or antibody fragment to polypeptides in a control sample. The comparison with the control sample allows the conclusion whether or not the test individual is affected with the disease. The control sample is preferably from a healthy control individual. As used herein, a healthy individual refers to an individual who is confirmed not to be afflicted with the particular disease that is to be diagnosed. For example, whereas the disease to be diagnosed is characterized by an abnormal reduction of Man6P residues present on lysosomal enzymes, a healthy individual will exhibit a normal amount of appropriately labeled lysosomal enzymes. If the control sample is derived from a healthy individual, a reduced degree of binding of the antibody or antibody fragment to polypeptides in the sample of the test individual relative to the degree of binding of the antibody or antibody fragment to polypeptides in the control sample is indicative that the test individual is affected with the disease.

Alternatively, samples derived from individuals who are diagnosed to be affected with the disease, may be used as positive control. Here the degree of binding of the antibody or antibody fragment to the sample which essentially corresponds to the degree of binding observed in the positive control sample from the individual affected by the disease indicates that the test individual is also afflicted with the disease. It will be appreciated by the person skilled in the art that distinct parameters may be adapted according to routine methods in order to optimize the method according to the invention. These parameters will include the absolute amount of the antibody or antibody fragment which is to be applied to the test sample as well as the conditions of binding of the antibody or antibody fragment to the antigen in the test sample. Said parameters will amongst other depend from the nature of the cell sample which is subjected to the test and the specific antibody or antibody fragment used.

The present invention therefore also relates to the use of an antibody or antibody fragment of the invention for the diagnosis of a disease which is characterized by an absent or reduced modification (labeling) of molecules with Man6P residues.

The antibodies and antibody fragments of the present invention are also suitable for use in screening assays for identifying compounds which are capable of modulating, i.e. enhancing or inhibiting, the binding of the antibody and antibody fragment of the invention to a molecule which contains one or more Man6P moieties. Compounds which modulate the binding of molecules containing one or more Man6P moieties, for example, lysosomal proteins, to their receptors or antibodies may be useful in enzyme replacement therapy and as molecular tools in research of the mechanisms underlying the synthesis and sorting of lysosomal proteins in the cell. In such a screening assay, an antibody or antibody fragment of the invention is incubated with a molecule comprising one or more Man6P moieties in the presence of a candidate compound which is to be examined with respect to potential effects on the binding reaction. The resulting binding complexes between the antibody or antibody fragment and the Man6P-containing molecule are detected according to methods known in the art.

The results of the detection are then compared with a control reaction. As a suitable control, one could use a binding reaction between the same antibody or antibody fragment and the same target molecule in the absence of any modulatory compound. Of particular interest is the identification of compounds which lead to an increased binding of the antibody/antibody fragment to the target molecule. These compounds should affect the activity/selectivity/efficiency of the GlcNac-1-phosphotransferase or GlcNac phosphodiesterase resulting in a higher number of Man6P residues per molecule. These compounds could potentially enhance the binding of Man6P-labeled proteins to their natural receptor proteins in vivo which renders them useful, for example, in therapeutic approaches for treating diseases which are characterized by a deficiency of single Man6P-containing proteins. In particular, these compounds might prove useful in enzyme replacement therapy for enhancing the uptake of exogenous lysosomal proteins which are administered to a patient.

Candidate compounds which may have an effect on the formation of binding complexes may encompass different chemical classes. Typically, these compounds are organic molecules, such as small organic molecules having a molecular weight of between 50 and 5000 Da. Candidate compounds may be obtained from a variety of different sources including libraries of synthetic or natural compounds. For example, numerous means are available for random and directed synthesis of organic compounds and biomolecules, including expression of randomized oligonucleotides and oligopeptides. Alternatively, libraries of natural compounds in the form of bacterial, fungal, plant and animal extracts are readily available in the art.

### EXAMPLES

### EXAMPLE 1: Preparation of PMP-BSA and Immunisation

Bovine serum albumin conjugated with pentamannose 6-phosphate (PMP-BSA) was prepared from the phosphomannan fraction of the yeast *Hansenula holstii* (kindly provided by Dr. M. Slodki; United States Department of Agriculture, Northern Regional Research Center, Peoria, IL). The general procedure of conjugating PMP with bovine serum albumin has previously been described in Braulke, T. et al. (1987), J. Cell Biol. 104, 1735-1742. The ratio of coupled ligand per mole of bovine serum albumin was approximately 20:1 as determined by anthrone reaction.

Rabbits were immunised and boostered with each 0.15 mg PMP-BSA neoglyoconjugate. Ten days after booster injection, the rabbits were sacrificed by exsanguination under anesthesia, and bone marrow was isolated from the femur. All procedures were in conformity with the institutional guidelines in the animal facility of the University Medical Center Hamburg-Eppendorf.

### EXAMPLE 2: Phage-Display

A library of phage-displayed single-chain Fv (scFv) antibodies was generated using RNA isolated from bone marrow cells of the immunised rabbits as starting material. The procedure is described, for example, in Barbas et al., Phage-Display: A Laboratory Manual, Cold Spring Harbor Laboratory Press, New York (2001) Protocol 9.7, pages 9.79-9.89. The harvested bone marrow cells were lysed, and RNA was isolated using the PeqGold Trifast kit (PeqLab GmbH, Germany) according to the instructions of the supplier. Generation and display of the scFv antibodies on M13 phage as pIII fusion proteins was achieved using the protocol and primers published in Phage-Display: A Laboratory Manual. Cold Spring Harbor Laboratory Press, New York (2001), and the pComb3XSS phagemid vector. The phagemid vector pComb3 XSS was kindly provided by Dr. C. F. Barbas III, Scripps Research Institute, La Jolla, California, USA. Amplification of antibody variable light and heavy regions using the primers described by Barbas et al. (see above), the assembly of a polynucleotide sequence encoding the single-chain antibody fragment by overlap-extension PCR and subsequent cloning of the polynucleotide sequence into the vector pComb3XSS introduced an OmpA leader peptide and an artificial linker sequence connecting the C-terminus of the light chain with the N-terminus of the heavy chain Fv into the expressed protein.

The initial library contained 5 x 10⁶ members, and colony PCR indicated that 19 out of 20 colonies contained an antibody gene of the expected size. Phage-displayed scFv were enriched by 5 rounds of phage panning on PMP-BSA which was immobilized at 1 µmol/cup in sodium carbonate buffer pH 9.2. As input for the panning procedure approximately 10¹¹ phages were added, and after 2 h incubation at 37°C, the supernatant was removed and unbound phage was washed away (1^{st} round 5x washing; 2^{nd} round 10x, 3^{rd} and 4^{th} round 12x, and 5^{th} round 15x). Bound phage was eluted with 0.1 M glycine/HCl buffer pH 2.2 containing 0.1% BSA. Titration of the output after the 5^{th} round of panning revealed 1.8 x 10⁶ phages/ml which were amplified and subsequently used in an ELISA.

The reactivity of the enriched polyclonal phage was tested in an ELISA checkerboard titration against different concentrations of PMP-BSA (3-100 pmol/cup calculated for PMP concentration). At 50 pmol/cup reactivity of the polyclonal phage was observed selectively for PMP-BSA but not for BSA. BstOI (Promega GmbH, Germany) restriction digest of 20 colony PCR products indicated the presence of 7 different clones (4 x type A, 10 x type B, 2 x type C and 1 x types D-G). Twelve colonies representing the different types of sequences (5 x type B, 2 x type A, 1 x each of types C-G) were selected for phage production and 1:3 (v/v) dilutions of phage were tested in ELISA against PMP-BSA. Both phages containing scFv of sequence type A (designated scFv M6P-1), but none of the other reacted. The primary structure of scFv M6P-1 was deduced from the cloned cDNA sequence.

### EXAMPLE 3: Expression and purification of soluble scFv

To transfer the isolated polynucleotide sequence encoding the scFv from pComb3XSS into the expression vector pSJF8, a PCR was performed using primers which allowed the cloning of the PCR product into the EcoRI and BamHI restriction sites of the vector pSJF8, see MacKenzie, C.R. (2000), Methods Enzymol. 312, 205-216. These primers replaced the HA-tag of pComb3XSS and introduced a C-terminal c-myc tag into the protein. Cloning into these restriction sites furthermore added a His-tag to the expressed protein for purification (see figure 4). The following PCR conditions were used: 3 min, 94 °C; 30x 30 s, 94°C, 60°C, 30 s, 72°C, 1 min; 72°C, 10 min. The following primers were used: 5'-GGGGGGAATTCATGAAAAAGACAGCTATCGCGATTGC-3' (SEQ ID NO:11; EcoRI site underlined, forward primer) and 5'-GGGGGATCCCTTCAAATCTTCCTCACTGATTAGCTTCTGTTCAGATCTTGAGGAGACGGTG-ACCAGGGT-3' (SEQ ID NO:12; BamHI site underlined, reverse primer). The amplified DNA was cloned into the expression vector pSJF8. Following transformation of *E*. *coli* TG-1 by electroporation and expression at 24°C over 72 h post IPTG induction (final concentration 1 mM), cells were harvested by centrifugation (7000x g, 4°C, 30 min) and resuspended in cold MAPS II elution buffer pH 6.8 (BioRad, Germany, 200 ml/100 g wet cells). Polymyxin B was added to a final concentration of 0.1 mg/ml. After incubation (4°C, 4 h, stirring), the cells were spun down and the supernatant collected. The supernatants of three extractions were combined and soluble scFv was purified by affinity chromatography (IMAC, 5 ml HiTrap cartridges, GE Healthcare) followed by gel filtration on Superdex HR 75 (HiLoad 16/60 Superdex 75 pg, GE Healthcare) from the combined supernatants as described in Anand, N.N. et al. (1991), J. Biol. Chem. 266, 21874-21879. The yield of scFv was 8 mg per L culture, as determined using a composition-derived molar extinction coefficient ε = 57070 leading to A₂₈₀ 1.0 = 0.5 mg/ml.

### EXAMPLE 4: Enzyme-linked Immunosorbent Assay (ELISA)

ELISA was performed according to the protocol published in Phage-Display: A Laboratory Manual, Cold Spring Harbor Laboratory Press, New York (2001), protocol 19.8, pages 19.29-19.31. For ELISA using monoclonal phage or soluble scFv, PMP-BSA was immobilized by addition of 2.25 pmol neoglycoconjugate (45 pmol PMP ligand, solution in 50 mM carbonate buffer, pH 9.2) to each cup of the ELISA plates (MaxiSorp, Nunc) and overnight incubation at 4°C. Phages were detected using an anti-M13 monoclonal antibody (mAb) HRP-conjugate (GE Healthcare, Freiburg, Germany) and diammonium 2,2'-azino-bis(3-ethyl-benzothiazoline-6-sulfonate (AzBTS-(NH₄)₂, Sigma-Aldrich) as substrate. For scFv-ELISA against PMP-BSA, purified scFv was added at a starting concentration of 5 µg/ml. Bound scFv was detected by incubation with anti-c-myc mAb (gift from Dr. C.R. MacKenzie, NRC of Canada, Ottawa, Canada) and HRP-conjugated goat-anti-mouse IgG H+L (Dianova, Hamburg, Germany).

For ELISA inhibition studies, 45 pmoles PMP-BSA were immobilized on ELISA plates. Monomeric scFv M6P-1 was freshly prepared by gelfiltration (see above). Antibody fragment scFv (0.6 µg/ml final concentration) was preincubated for 1 h at 37°C with or without 0.01 to 100 mM of mannose, glucose, glucose-6-phospate (Glc6P), Fru1P, or Man6P. 50 µl scFv M6P-1 were added to PMP-BSA (45 pmol/cup ligand concentration). Bound scFv M6P-1 was quantified after incubation (i) with an antibody directed against the c-myc tag of the antibody scFv M6P-1 and (ii) a HRP-conjugated goat-anti-mouse H/L antibody. The DAB reaction was measured spectrophotometrically at OD405. All data were measured in quadruplicates and the standard deviation from the mean is indicated. The data were analyzed using Origin version 6.0 software (MicroCal, Northampton, USA) and fitted to a 4 parameter logistic function. The data obtained for inhibition by Man6P and Fru1P were globally fitted to the four replicate data sets, each separately.

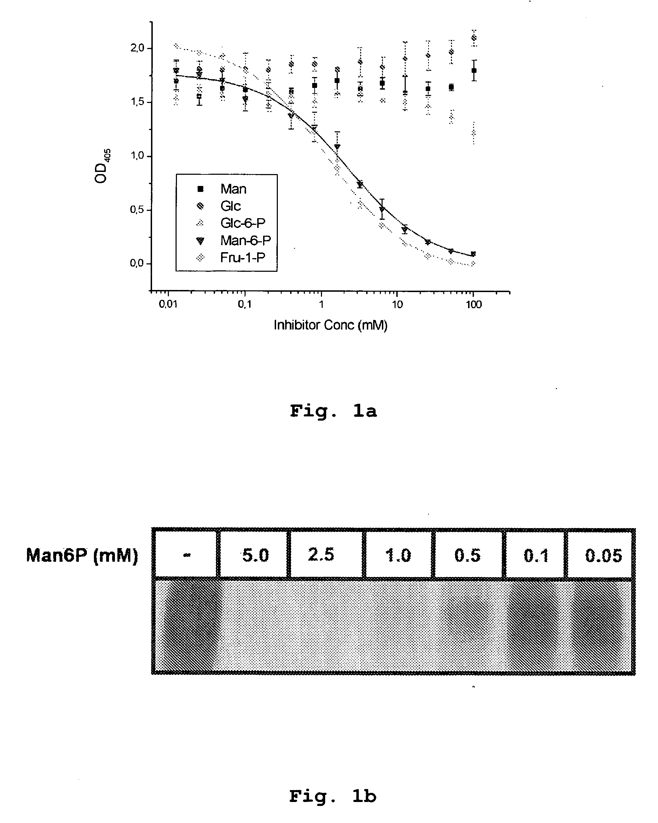

The results of the ELISA inhibition studies are shown in Fig. 1a. Man6P was able to inhibit the reactivity of scFv M6P-1 with PMP-BSA, and 50 % inhibition was observed at 2.3 mM Man6P. Also, β-D-fructopyranose-1-phosphate (Fru1P), which binds to the Man6P receptor due to structural similarities to Man6P (Olsson, 1999), inhibited scFv M6P-1 binding to PMP-BSA with an IC50 value of 1.1 mM. Neither mannose, glucose or glucose 6-phosphate (Glc6P) inhibited the binding of scFv M6P-1 to PMP-BSA even at high concentrations.

### EXAMPLE 5: Immobilisation of scFv M6P-1 on beads and Immunoprecipitation of [¹²⁵I]ASA

Monomeric or dimeric scFv M6P-1 (about 1 mg/ml in PBS, pH 7.2) isolated by gel filtration (see above) was chemically immobilized to AminoLink Plus Coupling gel beads (Pierce, USA) following the suppliers instructions (1 mg scFv M6P-1 was coupled to 2 ml of settled beads). Human arylsulfatase A (ASA)(kindly provided by Dr. T. Dierks; Institute for Biochemistry, University of Bielefeld, Germany) was purified by affinity chromatography from media of BHK cells which overexpress the enzyme as described by Sommerlade et al. (1994) Biochem. J. 297, 123-130 and iodinated with IODO-GEN (Pierce) according to the manufacturer's instructions yielding [¹²⁵I] ASA with a specific activity of 2µCi/µg. [¹²⁵I]ASA (400,000 cpm) was incubated with 50 µl scFv M6P-1 conjugated beads in 10 mM phosphate buffered saline pH 7.4 containing 0.05 % Triton X-100 in a final volume of 0.5 ml in the absence or presence of increasing concentrations of Man6P for 10 h at 4°C. Subsequently, the beads were spun down at 1,200 x g for 20 sec, and the supernatant was removed. The beads were washed three times with ice-cold binding buffer, twice with 1 mM phosphate buffered saline pH 7.4, and solubilized in SDS sample buffer. After removal of the beads, the supernatants were separated by SDS-PAGE (10% acrylamide), and the [¹²⁵I]ASA was visualized by autoradiography using Kodak films. The intensity of the [¹²⁵I]-labeled polypeptides was evaluated by densitometry. Alternatively, the [¹²⁵I]-labeled polypeptides were excised from the dried gel and counted in a γ-counter (Wizard 1470, Wallac, Turku, Finland).

The results are shown in Fig. 1b. About 20-25 % of total offered [¹²⁵I]-ASA bound specifically to the scFv M6P-1 conjugated beads . As can be seen in Fig. 1b, Man6P in a concentration of more than 2.5 mM completely inhibited the binding of [¹²⁵I]-ASA to the antibody beads, whereas Glc6P (5 mM) showed no effect on binding. After densitometric evaluation of the autoradiographs or determination of the radioactivity in excised [¹²⁵I]-labeled bands a Ki value of 100 µM was calculated for Man6P, which is similar to Kd values determined for the 46 kDa and 300 kDa Man6P receptor. See Tong et al. (1989), J. Biol. Chem. 264, 7970-7975 (1989).

### EXAMPLE 6: Isothermal titration microcalorimetry

The comparison of IC₅₀ values allows the ranking of different ligands according to their relative affinities, however, the absolute affinities in terms of K_{d} can not be obtained. Therefore, isothermal titration microcalorimetry measurements using Man6P as a ligand were performed. Purified dimeric scFv M6P-1 obtained after gel filtration (both a monomer and a dimer fraction are obtained) was concentrated in Centriprep YM-10 (Millipore) to a final concentration of 2.7 mg/ml in PBS, pH 7.2 and dialysed for 1 h at 20°C against 500 ml of PBS. The same dialysis buffer was used for preparation of a 10 mM solution of Man6P (Na⁺ salt, Sigma-Aldrich). Microcalorimetric experiments were performed on an MCS isothermal titration calorimeter (Microcal Inc., Northampton, MA, USA). The microcalorimeter cell was filled with the antibody solution (volume = 1.5 ml) and Man6P in dialysis buffer loaded into the syringe of the microcalorimeter. Both solutions were thoroughly degassed prior to loading. After temperature equilibration, the ligand was injected into the cell in 4 µl portions and the evolved heat measured with the first injection (1 µl) not considered for data analysis. A total of 21 injections were performed with 8 min equilibration times between injections. Data were corrected for heat of dilution by measuring the same number of buffer injections and subtraction from the sample data set. Dissociation constants were determined using the MicroCal Origin version 2.9 analysis software and non-linear least squares curve fitting (1 set of binding sites).

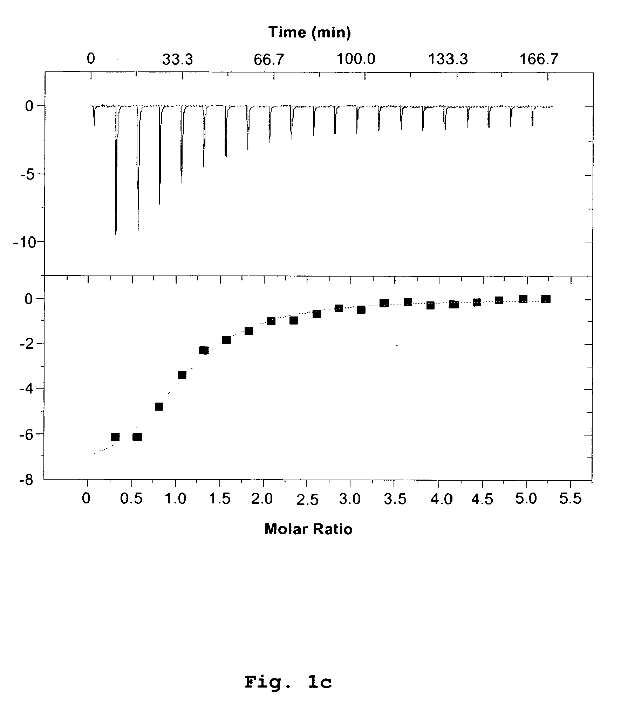

The results are shown in Fig. 1c. Microcalorimetry measurements yielded a Kd of 28 µM for the binding of scFv M6P-1 to Man6P.

### EXAMPLE 7: Purification of Man6P-containing lysosomal enzymes from media of overexpressing cells

To examine whether scFv M6P-1 can be used to enrich and purify lysosomal enzymes, recombinantly expressed human ASA and murine cathepsin D were purified from media of overexpressing cells. Serum-free media from BHK cells stably overexpressing human ASA (see Braulke, T. et al. (1990), J. Biol. Chem. 265, 6650-6655) or transiently expressing mouse cathepsin D (mCtsD) (see Partanen, S. et al. (2003), Biochem. J. 369, 55-62) were conditioned for 24 h. Aliquots of the media (0.25 ml) were mixed either with 0.25 ml of the other overexpressing cell line or with 0.25 ml of vector-transfected cells and incubated with 50 µl of scFv M6P-1 coupled beads for 16 h at 4° C. Afterwards the samples were centrifuged and the supernatant removed. Proteins bound to the scFv M6P-1 coupled beads were solubilized, and analysed by Western blotting using anti-hASA and anti-mCtsD polyclonal antibodies described previously (see Braulke, T. et al. (1990), J. Biol. Chem. 265, 6650-6655; Partanen, S. et al. (2003), Biochem. J. 369, 55-62.

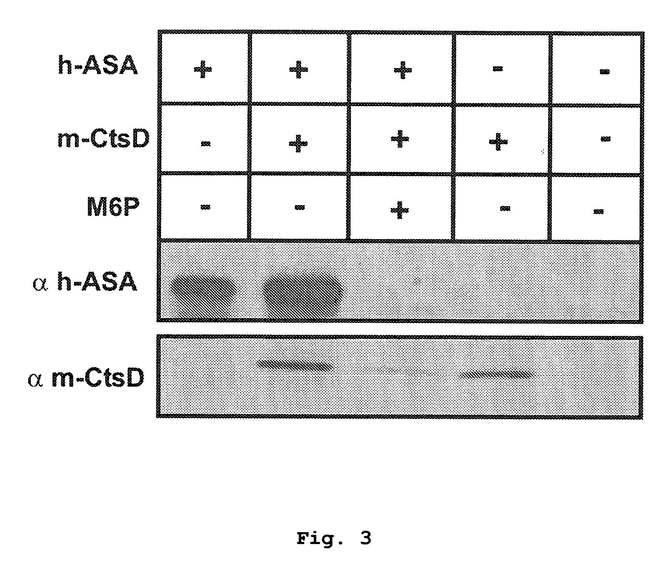

As shown in Fig. 3, both human ASA and murine cathepsin D could be isolated by immunoprecipitation from the media in a Man6P-dependent manner. No immunoreactive material could be precipitated from media of non-transfected cells.

### EXAMPLE 8: Applicability of scFv M6P-1 in the diagnosis of mucolipidosis type II

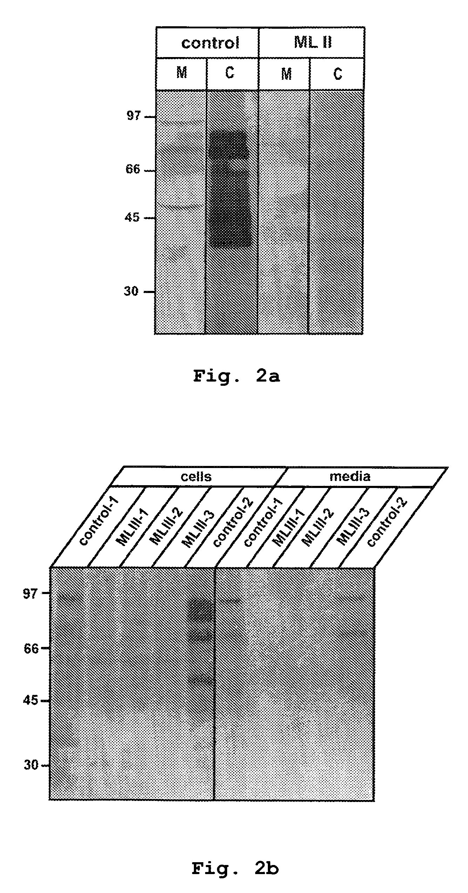

Extracts of cultured fibroblast cells and aliquots of media conditioned by these fibroblast cells were obtained from a healthy control individual and a patient with confirmed ML-II. The extracts were separated by SDS-PAGE and tested by Western blotting using biotinylated scFv M6P-1. In the extracts of control cells, six major immunoreactive bands of 83, 74, 64, 52, 45, and 40 kDa were detected (see Fig. 2a) representing most likely a group of mature proteolytically processed lysosomal enzymes equipped with long-life Man6P-residues. In the conditioned medium, the three most prominent immunoreactive bands exhibited molecular masses of 95, 75, and 52 kDa representing higher molecular mass precursor proteins that failed to bind to Man6P-receptors in the Golgi apparatus and comprise about 5-20% of total newly synthesized lysosomal enzymes during the time of conditioning.

In the cell extracts of the ML-II patient only weak signals were observed that differed in their molecular masses from immunoreactive bands in control cells. Furthermore, in the media of ML-II fibroblasts no immunoreactive material could be detected. Thus, the detection of Man6P-containing proteins in media or cultured cells by Western blotting using scFv M6P-1 leads to a clear distinction between Man6P-containing glycoproteins from cells of healthy individuals and ML-II patients and can thus be used as additional sensitive tool for diagnosis of ML-II.

Extracts of cultured fibroblast cells and aliquots of media conditioned by these fibroblast cells were obtained from two healthy control individual and three patients with confirmed ML-III. The extracts were separated by SDS-PAGE and tested by Western blotting using biotinylated scFv M6P-1. In the extracts of control cells, immunoreactive bands with similar molecular masses as shown in Fig. 2a were detected (see Fig. 2b) representing most likely a group of mature proteolytically processed lysosomal enzymes equipped with long-life Man6P-residues. In the conditioned medium of control cells, the three most prominent immunoreactive bands exhibited molecular masses of 95, 75, and 52 kDa representing higher molecular mass precursor proteins that failed to bind to Man6P-receptors in the Golgi apparatus and comprise about 5-20% of total newly synthesized lysosomal enzymes during the time of conditioning.

In the cell extracts of the ML-III patients only weak signals were observed that differed in their intensity and partially in their molecular masses from immunoreactive bands in control cells. Furthermore, in the media of ML-III fibroblasts no immunoreactive material could be detected. Thus, the detection of Man6P-containing proteins in media or cultured cells by Western blotting using scFv M6P-1 leads to a clear distinction between Man6P-containing glycoproteins from cells of healthy individuals and ML-III patients and can thus be used as additional sensitive tool for diagnosis of ML-III.

## Claims

1. Isolated antibody or antigen-binding fragment thereof which specifically binds to mannose 6-phosphate, wherein said antibody or fragment thereof comprises
(a) the complementarity-determining regions (CDRs) shown in SEQ ID NOs:1-6; or
(b) CDR sequences having at least 80% sequence identity to the CDRs shown in SEQ ID NOs:1-6;
wherein said antibody or antigen-binding fragment thereof does not show any significant binding to molecules other than mannose-6-phosphate or fructose-1-phosphate.

2. Antibody or antigen-binding fragment thereof according to claim 1 comprising
(a) the light chain variable region shown in SEQ ID NO:7 or a sequence having at least 80% sequence identity thereto; and
(b) the heavy chain variable region shown in SEQ ID NO:8 or a sequence having at least 80% sequence identity thereto.

3. Nucleic acid molecule encoding an antibody or an antigen-binding fragment according to any of claims 1-2.

4. Expression vector comprising a nucleic acid molecule according to claim 3.

5. Host cell comprising a nucleic acid molecule according to claim 3 or an expression vector according to claim 4.

6. Method for preparing an antibody or antibody fragment according to any of claims 1-2, wherein a host cell according to claim 6 is cultured under conditions which allow for the expression of the nucleic acid molecule encoding the antibody or the antigen-binding fragment thereof.

7. Method for purifying or concentrating a target molecule which comprises at least one mannose 6-phosphate residue from a sample, comprising the steps of
a) contacting an antibody or antibody fragment of any of claims 1-2 with said sample under conditions which allow for the formation of binding complexes comprising the antibody or antibody fragment and the target molecule;
b) separating the binding complexes from the sample, thereby purifying or concentrating the target molecule.

8. Method for identifying a target molecule which comprises at least one mannose 6-phosphate residue in a sample, comprising the steps of
a) contacting an antibody or antibody fragment of any of claims 1-2 with said sample under conditions which allow for the formation of binding complexes comprising the antibody or antibody fragment and the target molecule;
b) detecting binding complexes in the sample, thereby identifying the target molecule.

9. Method of claim 7 or 8, wherein the antibody or antibody fragment is immobilized on a solid support.

10. Method of any of claims 7-9, wherein the target molecule is a lysosomal enzyme.

11. Method for diagnosing mucolipidosis type II or mucolipidosis type III, said method comprising the steps of
a) contacting an antibody or antibody fragment of any of claims 1-2 with a cell sample from an individual who is suspected of being afflicted with said disease under conditions which allow for the formation of binding complexes comprising the antibody or antibody fragment and a molecule which comprises at least one mannose 6-phosphate residue;
b) determining the degree of binding of the antibody or antibody fragment to the molecules of the sample,
c) comparing said degree of binding of the antibody or antibody fragment to molecules of the sample with the degree of the binding of the antibody or antibody fragment to molecules in a control,
wherein the comparison to the control sample allows the conclusion whether or not the test individual is afflicted with the disease.

12. Method of claim 11, wherein the control sample is from a healthy control individual, and a reduced degree of binding of the antibody or antibody fragment to polypeptides in the sample of the test individual indicates that said test individual is afflicted with the disease.

13. Method of claim 11, wherein the control sample is from a control individual who is afflicted mucolipidosis type II or mucolipidosis type III, and a degree of binding which corresponds to the degree of binding observed in the control sample from the diseased control individual indicates that the test individual is afflicted with the disease.

14. Antibody or antigen-binding fragment according to any of claims 1-2 for use in medicine.

## Patentansprüche

1. Isolierter Antikörper oder Antigen-bindendes Fragment davon, der/das spezifisch an Mannose-6-Phosphat bindet, wobei der Antikörper oder das Fragment davon folgendes umfasst:
(a) die in SEQ ID NO:1-6 gezeigten komplementaritätsbestimmenden Regionen (complementarity-determining regions; CDRs), oder
(b) CDR-Sequenzen, die mindestens 80% Sequenzidentität zu den in SEQ ID NO:1-6 gezeigten CDRs aufweisen,
wobei der Antikörper oder das Antigen-bindende Fragment davon keine signifikante Bindung an Moleküle zeigen, die nicht Mannose-6-Phosphat oder Fruktose-1-Phosphat sind.

2. Antikörper oder Antigen-bindendes Fragment davon nach Anspruch 1, umfassend:
(a) die in SEQ ID NO:7 gezeigte variable Region der leichten Kette oder eine Sequenz, die mindestens 80% Sequenzidentität zu dieser aufweist; und
(b) die in SEQ ID NO:8 gezeigte variable Region der schweren Kette oder eine Sequenz, die mindestens 80% Sequenzidentität zu dieser aufweist.

3. Nukleinsäuremolekül, das für einen Antikörper oder ein Antigen-bindendes Fragment davon nach einem der Ansprüche 1-2 kodiert.

4. Expressionsvektor, der ein Nukleinsäuremolekül nach Anspruch 3 umfasst.

5. Wirtszelle, die ein Nukleinsäuremolekül nach Anspruch 3 oder einen Expressionsvektor nach Anspruch 4 umfasst.

6. Verfahren zur Herstellung eines Antikörpers oder eines Antikörper-Fragments nach einem der Ansprüche 1-2, wobei eine Wirtszelle nach Anspruch 6 unter Bedingungen kultiviert wird, welche die Expression des Nukleinsäuremoleküls, das den Antikörper oder das Antigen-bindende Fragment davon kodiert, ermöglichen.

7. Verfahren zur Aufreinigung oder Konzentrierung eines Zielmoleküls, das mindestens einen Mannose-6-Phosphatrest umfasst, aus einer Probe, bei dem man:
(a) einen Antikörper oder ein Antikörper-Fragment nach einem der Ansprüche 1-2 mit der Probe unter Bedingungen in Kontakt bringt, die die Bildung von Bindungskomplexen, welche den Antikörper oder das Antikörper-Fragment und das Zielmolekül umfassen, ermöglichen;
(b) die Bindungskomplexe von der Probe trennt, wodurch das Zielmolekül aufgereinigt oder aufkonzentriert wird.

8. Verfahren zur Identifizierung eines Zielmoleküls, das mindestens einen Mannose-6-Phosphatrest umfasst, in einer Probe, bei dem man:
(a) einen Antikörper oder ein Antikörper-Fragment nach einem der Ansprüche 1-2 mit der Probe unter Bedingungen in Kontakt bringt, die die Bildung von Bindungskomplexen, welche den Antikörper oder das Antikörper-Fragment und das Zielmolekül umfassen, ermöglichen;
(b) die Bindungskomplexe in der Probe nachweist, wodurch das Zielmolekül identifiziert wird.

9. Verfahren nach Anspruch 7 oder 8, wobei der Antikörper oder das Antikörper-Fragment auf einem festen Träger immobilisiert ist.

10. Verfahren nach einem der Ansprüche 7-9, wobei das Zielmolekül ein lysosomales Enzym ist.

11. Verfahren zur Diagnose von Mukolipidose Typ II oder Mukolipidose Typ III, bei dem man:
(a) einen Antikörper oder ein Antikörper-Fragment nach einem der Ansprüche 1-2 mit einer Zellprobe von einem Individuum, von dem angenommen wird, dass es unter dieser Krankheit leidet, unter Bedingungen in Kontakt bringt, welche die Bildung von Bindungskomplexen, die den Antikörper oder das Antikörper-Fragment und ein Molekül umfassen, welches mindestens einen Mannose-6-Phosphatrest umfasst, ermöglichen;
(b) das Ausmaß der Bindung des Antikörpers oder des Antikörper-Fragments an die Moleküle der Probe bestimmt;
(c) das Ausmaß der Bindung des Antikörpers oder des Antikörper-Fragments an Moleküle der Probe mit dem Ausmaß der Bindung des Antikörpers oder des Antikörper-Fragments an Moleküle in einer Kontrolle vergleicht;
wobei der Vergleich mit der Kontrollprobe die Schlussfolgerung erlaubt, ob das Testindividuum an der Krankheit leidet oder nicht.

12. Verfahren nach Anspruch 11, wobei die Kontrollprobe von einem gesunden Individuum stammt, und wobei ein verringertes Ausmaß der Bindung des Antikörpers oder des Antikörper-Fragments an Polypeptide in der Probe zeigt, dass das Testindividuum an der Krankheit leidet.

13. Verfahren nach Anspruch 11,' wobei die Kontrollprobe von einem Kontrollindividuum stammt, das an Mukolipidose Typ II oder Mukolipidose Typ III leidet, und wobei ein Ausmaß der Bindung, welches dem in der Kontrollprobe von dem erkrankten Kontrollindividuum beobachteten Ausmaß der Bindung entspricht, zeigt, dass das Testindividuum an der Krankheit leidet.

14. Antikörper oder Antigen-bindendes Fragment davon nach einem der Ansprüche 1-2 zur Verwendung in der Medizin.

## Revendications

1. Anticorps isolé ou fragment de liaison antigène de celui-ci, liant spécifiquement le mannose-6-phosphate, l'anticorps ou le fragment de celui-ci comprenant
les régions déterminant une complémentarité (CDR) montrées dans les séquences ID N° 1 à 6 ; ou
des séquences CDR ayant au moins 80% d'identité de séquence avec les CDR montrées dans les séquences ID N° 1 à 6,
**caractérisé en ce que** l'anticorps ou le fragment de liaison antigène de celui-ci ne montre pas de liaison significative à des molécules autres que le mannose-6-phosphate ou le fructose-1-phosphate.

2. Anticorps ou fragment de liaison antigène selon la revendication 1, comprenant
a) la région variable de chaîne légère montrée dans la séquence ID n° 7 ou une séquence ayant au moins 80% d'identité de séquence par rapport à celle-là ; et
b) la région variable de chaîne lourde montrée en séquence ID n0° 8 ou une séquence ayant au moins 80% d'identité de séquence par rapport à celle-là.

3. Molécule d'acide nucléique encodant un anticorps ou un fragment de liaison antigène selon la revendication 1 ou 2.

4. Vecteur d'expression comprenant une molécule d'acide nucléique selon la revendication 3.

5. Cellule hôte comprenant une molécule d'acide nucléique selon la revendication 3 ou un vecteur d'expression selon la revendication 4.

6. Procédé pour préparer un anticorps ou un fragment anticorps selon la revendication 1 ou 2, où la cellule hôte selon la revendication 5 est cultivée sous des conditions permettant à l'expression de la molécule d'acide nucléique de coder l'anticorps ou le fragment de liaison antigène de celui-ci.

7. Procédé de purification ou de concentration d'une molécule cible comprenant au moins un résidu de mannose-6-phosphate d'un échantillon, le procédé comprenant les étapes
a) mettre en contact avec l'échantillon un anticorps ou un fragment d'anticorps selon la revendication 1 ou 2 sous les conditions qui permettent la formation de liaisons complexes comprenant l'anticorps ou un fragment d'anticorps et la molécule cible ;
b) séparer les complexes de liaison de l'échantillon, en purifiant ou concentrant la molécule cible.

8. Procédé d'identification d'une molécule cible comprenant au moins un résidu de mannose-6-phosphate dans un échantillon, le procédé comprenant les étapes
a) mettre en contact avec l'échantillon un anticorps ou un fragment d'anticorps selon la revendication 1 ou 2 sous les conditions qui permettent la formation de liaisons complexes comprenant l'anticorps ou un fragment d'anticorps et la molécule cible ;
b) détecter des complexes de liaison dans l'échantillon, et identifier la molécule cible par cela.

9. Procédé selon la revendication 7 ou 8, **caractérisé en ce que** l'anticorps ou le fragment d'anticorps est immobilisé sur un support solide.

10. Procédé selon l'une des revendications 7 à 9, la molécule cible étant une enzyme lysosomale.

11. Procédé de diagnose de la mucolipidose de type II ou de la mucolipidose de type III, le procédé comprenant les étapes
a) mettre en contact un anticorps ou un fragment d'anticorps selon la revendication 1 ou 2 avec un échantillon de cellules d'un individu dont on suppose qu'il est infecté de cette maladie sous les conditions qui permettent la formation d'un complexe de liaison comportant l'anticorps ou le fragment d'anticorps et une molécule comportant au moins un résidu de mannose-6-phosphate ;
b) déterminer le degré de liaison de l'anticorps ou du fragment d'anticorps aux molécules de l'échantillon,
c) comparer le degré de liaison de l'anticorps ou du fragment d'anticorps à des molécules dans un contrôle,
où la comparaison à l'échantillon de contrôle permet la conclusion si l'individu de teste est infecté de la maladie ou non.

12. Procédé selon la revendication 11, où l'échantillon de contrôle est originaire d'un individu de contrôle de bonne santé, et un degré réduit de liaison de l'anticorps ou du fragment d'anticorps à des polypeptides dans l'échantillon de l'individu de teste indique que cet individu de teste est infecté de la maladie.

13. Procédé selon la revendication 11, où l'échantillon de contrôle est originaire d'un individu de contrôle infecté de la mucolipidose de type II ou de la mucolipidose de type III, et un degré de liaison qui correspond au degré de liaison observé dans l'échantillon de contrôle de l'individu de contrôle infecté indique que l'individu de test est infecté de cette maladie.

14. Anticorps ou fragment de liaison antigène selon la revendication 1 ou 2 pour application en médecine.
